# EUROPEAN PATENT SPECIFICATION

(11) **EP 0 394 687 B1**
(45) Date of publication and mention of the grant of the patent: **03.08.1994**
(21) Application number: 90105856.0
(22) Date of filing: 28.03.1990
(51) Int. Cl.: A61M 1/00, A61L 2/16

(54) **Suction drainage infection control system**
Absaugvorrichtung mit einer Einrichtung zur Infektionsvermeidung
Dispositif de drainage par aspiration et moyens limitant les risques d'infection

(30) Priority: 30.03.1989 US 330552; 27.12.1989 US 457442
(43) Date of publication of application: 31.10.1990
(73) Proprietor: ABBOTT LABORATORIES, Abbott Park, Illinois 60064-3500 (US)
(72) Inventor: Bryant, Peter L., Lake Forest, Illinois 60045 (US); Oswald, Timothy J., Lincolnshire, Illinois 60069 (US); Grabenkort, Richard W., Barrington, Illinois 60010 (US); Tripp, Edward S., Park City, Illinois 60085 (US)
(74) Representative: Modiano, Guido, Dr.-Ing.

(56) References cited:
- EP-A- 0 072 174
- EP-A- 0 138 427
- EP-A- 0 173 450
- WO-A-84/04036
- WO-A-87/00439
- FR-A- 2 380 069
- US-A- 3 768 478
- US-A- 3 938 540

## Description

### BACKGROUND OF THE INVENTION

Suction drainage systems having a connection from a rigid container or a flexible liner to the body of a patient and a connection from the container or liner to a suction source have been widely utilized in hospitals. These systems collect waste from surgical and other patients in a disposable container or flexible liner having an integral lid or cover. The waste being collected often is highly infectious and often subject to exposure caused by spills or a failure of the suction drainage system. Known from WO-A-87/00439 is a suction system for draining waste from a source as defined in the precharacterizing part of claim 1. Also known from US-A-3,938,540 is a vacuum-operated fluid collection system including a serially connected tandem system.

Accordingly, it is an object of the present invention to provide a suction drainage container infection control system.

It is a further object of the present invention to provide a suction drainage infection control system incorporating a germicide and a transfer system.

### SUMMARY OF THE INVENTION

The present invention is directed to a suction drainage infection control system as defined in the appended claims.

More particularly, the present invention is directed to a suction drainage infection control system that incorporates a germicide in a degradable or permeable package in a disposable rigid container or flexible liner.

This invention is also directed to a suction drainage infection control system having improved valves, and a multi-container transfer system.

The suction drainage infection control system of the present invention includes means for chemically treating the waste. The system can also include means for distributing germicide throughout the waste. Each suction drainage canister may be used alone or in series with one or more additional canisters.

The suction drainage canister infection control system of the present invention minimizes the risk of exposure for hospital personnel to infectious waste by decreasing the risk of infection and spills caused by failure to cap off full or partially full waste containers, accidental cap disconnection and liner breakage.

The suction drainage canister infection control system of the present invention promotes the safe handling of potentially infectious suction waste by exposing the collected waste to an effective germicidal agent that is capable of killing many types of bacteria and viruses at room temperature. The germicide is effective against HIV, hepatitis B, herpes simplex I, polio, adeno virus, and many other potentially infectious materials, and thus dramatically reduces the potential of cross-contamination between patients and minimizes the associated risk to health care workers.

### BRIEF DESCRIPTION OF THE DRAWINGS

The present invention will best be understood by reference to the following specification and claims taken in conjunction with the accompanying drawings. Figures 1-5 and the following description relating thereto set forth suction drainage systems illustrating aspects of the invention which are not encompassed by the appended claims, while figures 6 and 7 illustrate the invention defined in the appended claims. Reference will now be made to the drawings in which:

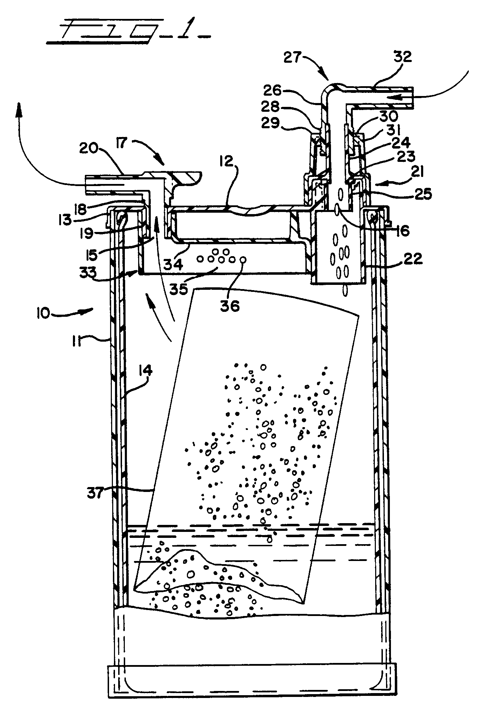

FIGURE 1 is a vertical section of a first embodiment while waste fluid is being drawn into the liner;

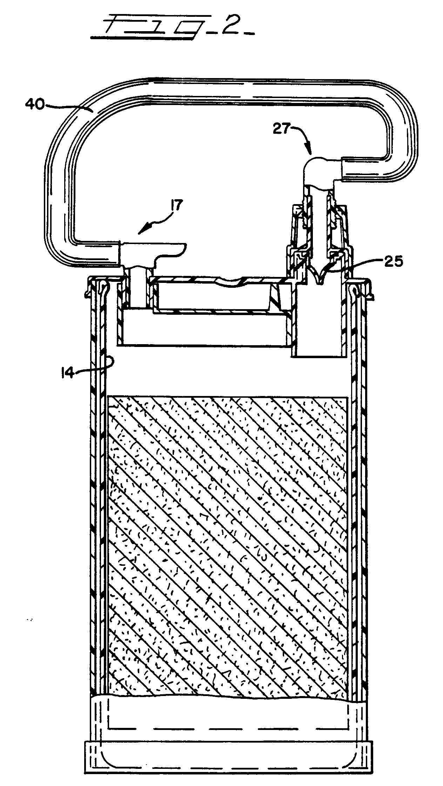

FIGURE 2 is a vertical section of the embodiment of FIGURE 1 after the liner is full and the germicide and/or absorbent composition has treated and/or solidified the waste;

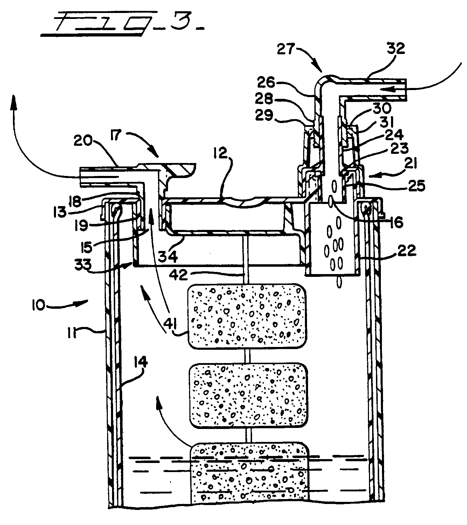

FIGURE 3 is a partial vertical section of a second embodiment that permits improved dispersion of the germicide and/or absorbent composition;

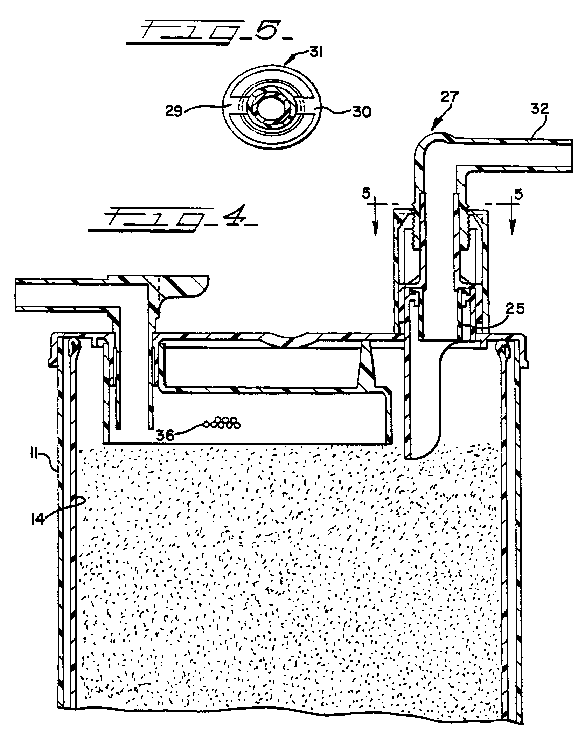

FIGURE 4 is a fragmentary vertical section of an embodiment that is adapted for use with a single canister;

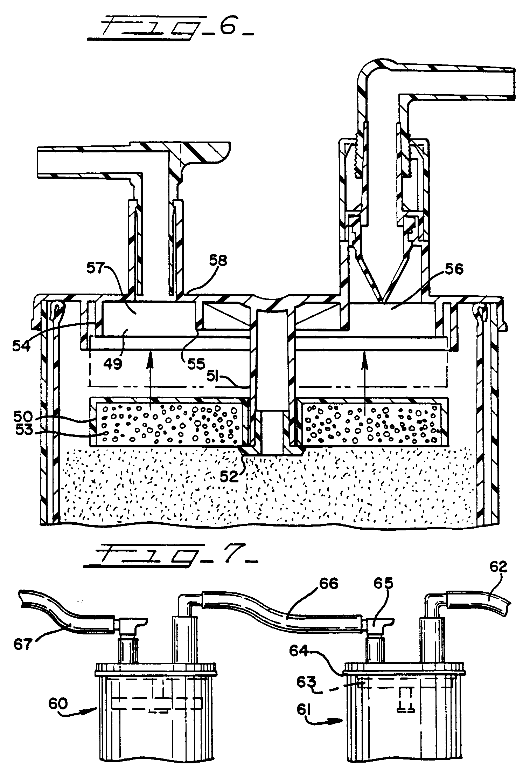

FIGURE 5 is a sectional view taken substantially as indicated by the line 5-5 of FIGURE 4;
FIGURE 6 is a fragmentary vertical section of an embodiment of the present invention that is adapted for use with two or more canisters connected in series; and
FIGURE 7 is a fragmentary diagrammatic view of two canisters of the present invention connected in series.

### DETAILED DESCRIPTION OF THE PREFERRED EMBODIMENTS

While the present invention is susceptible of embodiment in many forms, there is shown in the drawings and will hereinafter be described several presently preferred embodiments with the understanding that the present specification is to be considered as an exemplification of the invention, and is not intended to limit the invention to the specific embodiments illustrated.

Referring to the drawings, FIGURE 1 is a vertical section of a suction drainage container infection control system 10. The system 10 includes a cylindrical canister 11 that can be constructed of a relatively rigid plastic material and is open at the top and closed at the bottom. The canister itself is not contacted by the waste so it may be repeatedly used.

The canister 11 is adapted to receive a unitary structure comprising a canister lid 12 having a depending flange 13 for airtight engagement with the upper open end of the canister 11. Fused or otherwise secured to the underside of the lid 12 in a completely air-tight manner is a flexible liner 14. The lid 12 is preferably constructed from a relatively rigid plastic material, while the liner 14 is preferably constructed from a flexible thermoplastic material. Extending through the lid 12 are openings 15 and 16. Opening 15 contains a fitting 17 with a depending portion 18 which engages a depending flange 19 from the lid 12 in an airtight manner. The fitting 17 also includes an upper portion 20 that is adapted to be connected to a suction line (not shown).

A fitting 21 is inserted in opening 16 in an airtight manner. Fitting 21 includes a depending tubular portion 22 that extends above and below the surface of the lid 12 and helps to direct the incoming waste toward the bottom of the liner 14. Fitting 21 also has a shoulder 23 that supports a tubular upper portion 24 and a one-way double slit "duckbill" valve 25 that permits flow of waste into the liner 14 while preventing escape of waste from the liner. Other suitable duckbill valves are disclosed in U. S. Patent Nos. 3,822,720 and 3,901,272. The lower portion 26 of elbow 27 fits over upper arm 24. The lower portion 26 of elbow 27 has teeth 28 that are rotatably and/or straight push engaged by flanges 29 and 30 of a collar 31 that is engaged at its lower end by fitting 21. This secures elbow 27 to lid 12. Elbow 27 may be removed from the lid 12 by squeezing and/or rotating the upper portions of collar 31 that are between flanges 29 and 30.

A nonmechanical valve 33 is mounted in the lid 12. The nonmechanical valve 33 comprises a housing 34 that contains a polyethylene foam 35 containing swellable moisture sensitive particles 36 made of polymers or other suitable materials. A suitable nonmechanical valve is disclosed in published PCT Application No. WO 87/00439. This valve permits normal air flow through suction opening 15 until it becomes wet, whereupon the polymer particles swell to block air and waste flow.

The liner 14 contains a pouch 37 or other container constructed of a waste degradable or dissolvable material such as poly(vinylalcohol) or any other suitable waste degradable or dissolvable material or an open pore structure such as a tea bag-like structure that contains a germicide and/or an absorbent.

Representative suitable germicides include calcium hypochlorite, chlorinated trisodium phosphate, N-chlorosuccinimide, 1,3-dichloro-5,5-dimethylhydantoin, potassium dichloro-s-triazinetrione, sodium benzenesulfonchloramide, sodium hypochlorite, sodium p-toluene-sulfonchloramide, sodium dichloroisocyanurate, dihydrate, sodium dichloro-s-triazinetrione, p-sulfondichlor-amidobenzoic acid, p-toluenesulfondichloramide, trichloroisocyanuric acid, trichloromelamine, alcohols, formaldehyde, glutaraldehyde, hydrogen peroxide, iodine, quaternary ammonium compounds, paraacetic acid, paraformaldehyde, and phenols. Preferred germicides include 1,3-dichloro-5,5-dimethylhydantoin, potassium dichloro-s-triazinetrione, N-chlorosuccinimide, and sodium dichloroisocyanurate dihydrate.

Representative suitable absorbents include cellulose fibers, cross-linked polymeric salts, diatomaceous earth, dried clay, expanded silicate particulates, ground corncobs, perlite, silica gel, shredded polypropylene microfibers, sodium/calcium borosilicate glass, starch grafted sodium polyacrylate, thermally reticulated polyether polyurethane, and vermiculite.

As the liner is filled with waste, the pouch 37 disintegrates, opens, or dissolves and the germicide and/or absorbent is released. The germicide and/or absorbent will treat contaminants contained in the waste. In a preferred embodiment, the pouch also contains a swellable absorbent. Representative liquid absorbing and immobilizing packets are disclosed in U. S. Patent Nos. 4,748,069 and 4,749,600. the liner 14 can fill until the level of waste reaches nonmechanical valve 33. When the valve 33 becomes wet, the polymer particles swell to block air and waste flow out of the liner 14. With no vacuum being drawn through the liner, one-way valve 25 closes to prevent waste from flowing out of the liner through inlet opening 16.

FIGURE 2 is a vertical section of the embodiment of FIGURE 1 after the liner 14 has been filled with waste and the germicide and absorbent have been released into the waste. The absorbent swells and, upon removal the filled liner is semi-rigid or rigid. This tends to minimize the possibility of accidental rupture of or spills from the liner. One-way valve 25 is shown in its normal closed position. Tube 40 is also used to provide additional security against the possibility of accidental spills by securing elbow 27 to fitting 21. The lid and associated liner 14 may then be removed and discarded.

FIGURE 3 is a partial vertical section of a second embodiment in which individual packets 41 of germicide and/or absorbent are affixed to a flexible support 42 which is connected to the housing 34. As the waste level rises in the liner, each waste soluble or permeable packet 41 opens and releases its contents of germicide and/or absorbent into the waste and distributes the germicide and/or absorbent throughout the waste.

FIGURE 4 is a fragmentary vertical section of an embodiment that is adapted for use with a single canister. As waste enters the system 10 through the upper portion 32 of elbow 27, it passes through a one-way duckbill valve 25 into the liner 14 in canister 11. As the liner 11 fills with waste, the germicide and absorbent are released and the waste is treated. When the waste reaches the level of nonmechanical valve 33, the moisture-sensitive polymer particles 36 swell and shut off the suction pressure in the liner, which prevents any more waste from entering the system. The swollen polymer beads block air and waste flows out of the liner through opening 15 and the one-way duckbill valve 25 prevents waste from escaping through opening 16.

An additional feature of the system of the present invention is shown in FIGURE 5, which is a sectional view taken substantially as indicated by the line 5-5 of FIGURE 4. Collar 31 has a pair of opposing flanges 29 and 30 which engage the teeth on the elbow 27 to lock and retain the collar 31 on the fitting. Elbow 27 can be released by squeezing the opposing portions of collar 31 that are between flanges 29 and 30.

FIGURE 6 is a fragmentary vertical section of an embodiment of the present invention that is adapted for use with two or more canisters connected in series. In this embodiment, a float 50 is slidably mounted on post 51, which has a knob 52 at its lowermost portion to support the float in its lowermost position. The float 50 is packed with buoyant material 53 so that when the waste reaches a level in the liner 14 where it contacts the lower surface of the buoyant material 53 the float 50 rises until circular wall 54 is contacted by the upper surface of the float 50. Circular wall 54 forms with circular wall 55, an annular channel 49 through which opening 56 communicates with opening 57. The float 50 thus prevents the flow of waste into the liner 14. However, as seen from the drawings, suction is present and continues to draw in waste through valve 25. As a result of the suction, the waste will flow directly from inlet opening 56 to outlet opening 57 through circular channel 49. As shown in FIGURE 7, waste then can pass from one canister to another. This feature is referred to as the transfer system.

In FIGURE 7, there is shown a first container 60 and second container 61. Flexible tubing 62 is connected to means (not shown) for withdrawing waste from a patient. Container 61 is full of waste and float 63 is in sealing engagement in cap 64 so that waste passes directly to outlet means 65 and through flexible tubing 66 to canister 60 by means of suction being applied through tubing 67.

As stated herein, while FIGURES 1 through 7 are directed to a suction drainage infection control system having a flexible liner, the present invention is equally applicable to a rigid container containing germicide and a transfer system. Thus, as used herein, the term "container" is intended to include both disposable rigid containers and canisters having disposable flexible liners.

While the form of apparatus herein described constitutes preferred embodiments of the invention, it is to be understood that the invention is not limited to these precise forms of apparatus, and that changes may be made therein without departing from the scope of the invention that is defined in the appended claims.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. A suction system for draining waste from a source that comprises:
a container that comprises a canister (11, 61) that contains a flexible liner (14) and a lid (12) therefor to which the liner (14) is secured;
waste inlet means (16, 56) releasably secured to said lid (12) and having a one-way valve (25) that permits waste to flow only into said liner (14);
means (17-20, 57, 65) in said lid (12) attached to a suction source, and;
means (50-53, 63) in said lid (12) to stop suction from being applied in the container (11) when the waste in the liner (14) has reached a specified level;
characterized in that it further comprises means (37, 41, 42) to treat the waste in the liner (14), and means (57, 65) provided in said lid (12) for transferring suction and waste flow to additional containers (60), said means (50-53, 63) to stop suction from being applied in the container (11) when the waste in the liner (14) has reached a specified level comprising a float (50, 63) in the container that is movably mounted to the lid (12), said lid (12) and float (50, 63) being constructed and arranged to close off the suction and inlet means (16, 56) from the container (11, 61) when it reaches the specified level, but permit flow between said suction and inlet means (16, 56).

2. A suction system according to claim 1, characterized in that it further comprises at least one additional container (60) connected to said container (61).

3. A suction system according to claim 1, characterized in that the waste inlet means (16, 56) contains a one-way valve (25) that permits waste to flow only into the container (11, 61).

4. A suction system according to claim 1 or 3, characterized in that said one-way valve (25) is a double slit duckbill valve (25).

5. A suction system according to claim 1, characterized in that said container (11, 61) contains a waste dissolvable pouch or open pore structure (37, 41) having germicide therein.

6. A suction system according to claim 1, characterized in that the container (11, 61) contains a waste dissolvable pouch or open pore structure (41) having absorbent therein.

7. A suction system according to claim 1, characterized in that said float (50, 63) is slideably mounted on a post (51) having a knob (52) at its lowermost portion to support said float (50, 63) in a lowermost position.

8. Suction system according to claim 1 or 7, characterized in that said float (50, 63) is packed with buoyant material (53).

9. Suction system according to claims 1 and 7 or 8, characterized in that said container lid (12) has a pair of circular walls (54, 55) protruding therefrom within said container (11).

10. Suction system according to claim 9, characterized in that said float (50, 63) contacts said circular walls (54, 55) upon said container (11) becoming filled with waste.

11. Suction system according to claims 9 and 10, characterized in that it further comprises an annular channel (49) communicating said waste inlet means (56) with said means (57, 65) for transferring suction and waste flow to additional containers (60) attached to said suction source when said float (50, 63) contacts said circular walls (54, 55) upon said container (11) becoming filled with waste.

12. Suction system according to claims 2 and 10, characterized in that said means (57, 65) for transferring suction and waste flow to additional containers (60) is attached to said suction source through said additional container (60).

13. Suction system according to claim 12, further comprising a first tube (66) connecting said means (57, 65) to said additional container (60), and a second tube (67) connecting said additional container to said suction source.

## Patentansprüche

1. Eine Absaugvorrichtung für die Dränage von Abfallstoff einer Quelle, die Absaugvorrichtung umfassend:
einen Behälter, der einen Kanister (11,61) aufweist, der einen flexiblen Innensack (14) sowie hierfür eine Abdeckung (12) umfaßt, an welche der Innensack (14) angebracht ist;
ein Abfallstoffeinlaßmittel (16,56), das abnehmbar an der Abdeckung (12) angebracht ist und das ein Einwegventil (25) aufweist, das nur den Durchfluß von Abfallstoff in den Innensack (14) gestattet;
ein Mittel (17-20,57,65) in der Abdeckung (12), das an eine Absaugquelle angeschlossen ist, und;
ein Mittel (50-53,63) in der Abdeckung (12) zur Unterbrechung des am Behälter (11) angelegten Soges, wenn der Abfallstoff im Innensack (14) ein vorher bestimmtes Niveau erreicht hat;
dadurch gekennzeichnet, daß die Vorrichtung weiterhin ein Mittel (37,41,42) umfaßt, um den Abfallstoff im Innensack (14) zu behandeln, und ein in der Abdeckung (12) angeordnetes Mittel (57,65) zur Übertragung des Soges und des Abfallstoffdurchflusses auf zusätzliche Behälter (60), wobei das Mittel (50-53,63) zur Unterbrechung des am Behälter (11) angelegten Soges, wenn der Abfallstoff im Innensack (14) ein vorher bestimmtes Niveau erreicht hat, einen im Behälter angeordneten Schwimmer (50,63) umfaßt, der verschiebbar an der Abdeckung (12) montiert ist, wobei die Abdeckung (12) und der Schwimmer (50,63) derart ausgebildet und angeordnet sind, daß sie den Sog und das Einlaßmittel (16,56) zum Behälter (11,61) unterbrechen, wenn der Abfallstoff ein vorher bestimmtes Niveau erreicht hat, jedoch einen Durchfluß zwischen dem Sog und dem Einlaßmittel (16,56) gestatten.

2. Absaugvorrichtung nach Anspruch 1, dadurch gekennzeichnet, daß sie weiterhin zumindest einen zusätzlichen Behälter (60), der mit dem Behälter (61) verbunden ist, aufweist.

3. Absaugvorrichtung nach Anspruch 1, dadurch gekennzeichnet, daß das Abfallstoffeinlaßmittel (16,56) ein Einwegventil (25) umfaßt, das den Durchfluß des Abfallstoffs in den Behälter (11, 61) gestattet.

4. Absaugvorrichtung nach Anspruch 1 oder 3, dadurch gekennzeichnet, daß das Einwegventil (25) ein Doppelschlitz-Entenschnabelventil (25) ist.

5. Absaugvorrichtung nach Anspruch 1, dadurch gekennzeichnet, daß der Behälter (11,61) einen durch Abfallstoff auflösbaren Beutel oder eine offene Porenstruktur (37,41) anthält, worin sich ein Germizid befindet.

6. Absaugvorrichtung nach Anspruch 1, dadurch gekennzeichnet, daß der Behälter (11,61) einen durch Abfallstoff auflösbaren Beutel oder eine offene Porenstruktur (41) aufweist, worin sich ein Absorptionsmittel befindet.

7. Absaugvorrichtung nach Anspruch 1, dadurch gekennzeichnet, daß der Schwimmer (50,63) verschiebbar an einem Pfosten (51) montiert ist, der an seinem untersten Ende einen Knopf (52) aufweist, um den Schwimmer (50,63) in seiner untersten Stellung zu stützen.

8. Absaugvorrichtung nach Anspruch 1 oder 7, dadurch gekennzeichnet, daß der Schwimmer (50,63) mit einem Schwebstoff (53) gefüllt ist.

9. Absaugvorrichtung nach den Ansprüchen 1, 7 oder 8 dadurch gekennzeichnet, daß die Behälterabdeckung (12) ein Paar kreisförmiger Wände (54, 55) aufweist, die von der Abdeckung in den Behälter (11) hineinragen.

10. Absaugvorrichtung nach Anspruch 9, dadurch gekennzeichnet, daß der Schwimmer (50,63) mit den kreisförmigen Wänden (54, 55), bei Anfüllung des Behälters (11) mit Abfallstoff, in Berührung tritt.

11. Absaugvorrichtung nach den Ansprüchen 9 und 10, dadurch gekennzeichnet, daß diese weiterhin einen ringförmigen Kanal (49) aufweist, der das Abfallstoffeinlaßmittel (56) mit dem Mittel (57,65) zur Übertragung des Sogs und des Abfallstoffdurchflusses auf die zusätzlichen Behälter (60), die an die Absaugquelle angeschlossen sind, verbindet, wenn der Schwimmer (50,63) mit den kreisförmigen Wänden (54, 55), bei Anfüllung des Behälters (11) mit Abfallstoff, in Berührung tritt.

12. Absaugvorrichtung nach den Ansprüchen 2 und 10, dadurch gekennzeichnet, daß das Mittel (57,65) zur Übertragung des Sogs und des Abfallstoffdurchflusses auf die zusätzlichen Behälter (60) an die Absaugquelle über den zusätzlichen Behälter (60) angeschlossen sind.

13. Absaugvorrichtung nach Anspruch 12, die weiterhin ein erstes Rohr (66), das das Mittel (57, 65) mit dem zusätzlichen Behälter (60) verbindet, und ein zweites Rohr (67) aufweist, das den zusätzlichen Behälter mit der Absaugquelle verbindet.

## Revendications

1. Dispositif d'aspiration pour le drainage de déchets à partir d'une source, qui comprend :
un récipient comprenant une boîte (11, 61), qui contient une chemise souple (14) et un couvercle (12) pour celle-ci, auquel la chemise (14) est fixée;
un moyen d'entrée des déchets (16, 56) fixé de manière détachable audit couvercle (12) et possédant une vanne à une voie (25), qui permet aux déchets de s'écouler seulement dans ladite chemise (14);
un moyen (17-20, 57, 65) dans ledit couvercle (12) relié à une source d'aspiration, et;
un moyen (50-53, 63) dans ledit couvercle (12) pour arrêter l'aspiration appliquée dans le récipient (11) lorsque les déchets dans la chemise (14) ont atteint un niveau déterminé;
caractérisé en ce qu'il comprend en outre un moyen (37, 41, 42) pour traiter les déchets dans la chemise (14), et un moyen (57, 65), disposé dans ledit couvercle (12), pour transférer l'aspiration et l'écoulement des déchets à des récipients supplémentaires (60), ledit moyen (50-53, 63) pour arrêter l'aspiration appliquée dans le récipient (11) lorsque les déchets dans la chemise (14) ont atteint un niveau déterminé comprenant un flotteur (50, 63) dans le récipient, qui est monté de façon amovible avec le couvercle (12), ledit couvercle (12) et le flotteur (50, 63) étant construits et disposés pour fermer l'aspiration et le moyen d'entrée (16, 56) à partir du/dans le récipient (11, 61) lorsqu'il atteint le niveau déterminé, mais pour permettre l'écoulement entre ladite aspiration et le moyen d'entrée (16, 56).

2. Dispositif d'aspiration selon la revendication 1, caractérisé en ce qu'il comprend en outre au moins un récipient supplémentaire (60) relié audit récipient (61).

3. Dispositif d'aspiration selon la revendication 1, caractérisé en ce que le moyen d'entrée des déchets (16, 56) contient une vanne à une voie (25), qui permet aux déchets de s'écouler seulement dans le récipient (11, 61).

4. Dispositif d'aspiration selon la revendication 1 ou 3, caractérisé en ce que ladite vanne à une voie (25) est une vanne de type bec de canard à double fente (25).

5. Dispositif d'aspiration selon la revendication 1, caractérisé en ce que ledit récipient (11, 61) contient un sachet dissoluble dans les déchets ou une structure à pores ouverts (37, 41) à l'intérieur duquel/de laquelle se trouve un germicide.

6. Dispositif d'aspiration selon la revendication 1, caractérisé en ce que le récipient (11, 61) contient un sachet dissoluble dans les déchets ou une structure à pores ouverts (41) à l'intérieur duquel/de laquelle se trouve un absorbant.

7. Dispositif d'aspiration selon la revendication 1, caractérisé en ce que ledit flotteur (50, 63) est monté en pouvant coulisser sur un support (51) muni d'une protubérance (52) dans sa partie la plus basse pour supporter ledit flotteur (50, 63) dans une position la plus basse.

8. Dispositif d'aspiration selon la revendication 1 ou 7, caractérisé en ce que ledit flotteur (50, 63) est emballé avec un matériau flottable (53).

9. Dispositif d'aspiration selon les revendications 1 et 7 ou 8, caractérisé en ce que ledit couvercle (12) du récipient présente une paire de parois circulaires (54, 55) en saillie à partir de celui-ci, à l'intérieur dudit récipient (11).

10. Dispositif d'aspiration selon la revendication 9, caractérisé en ce que ledit flotteur (50, 63) touche lesdites parois circulaires (54, 55) du moment où ledit récipient (11) se remplit de déchets.

11. Dispositif d'aspiration selon les revendications 9 et 10, caractérisé en ce qu'il comprend en outre un canal annulaire (49) mettant en communication ledit moyen d'entrée des déchets (56) avec ledit moyen (57, 65) pour transférer l'aspiration et l'écoulement des déchets à des récipients supplémentaires (60), reliés à ladite source d'aspiration lorsque ledit flotteur (50, 63) touche lesdites parois circulaires (54, 55) du moment où ledit récipient (11) se remplit de déchets.

12. Dispositif d'aspiration selon les revendications 2 et 10, caractérisé en ce que ledit moyen (57, 65) pour transférer l'aspiration et l'écoulement des déchets à des récipients supplémentaires (60) est relié à ladite source d'aspiration par l'intermédiaire dudit récipient supplémentaire (60).

13. Dispositif d'aspiration selon la revendication 12, comprenant en outre un premier tube (66) reliant ledit moyen (57, 65) audit récipient supplémentaire (60), et un second tube (67) reliant ledit récipient supplémentaire à ladite source d'aspiration.
